# EUROPÄISCHE PATENTSCHRIFT

(11) **EP 2 864 757 B1**
(45) Veröffentlichungstag und Bekanntmachung des Hinweises auf die Patenterteilung: **11.03.2020**
(21) Anmeldenummer: 13732852.2
(22) Anmeldetag: 12.06.2013
(51) Int. Cl.: F02D 41/14, F02D 41/22, G01N 15/06, G01N 33/00

(54) **VERFAHREN ZUR FUNKTIONSKONTROLLE EINES SENSORS ZUR DETEKTION VON TEILCHEN UND SENSOR ZUR DETEKTION VON TEILCHEN**
METHOD FOR THE FUNCTIONAL CONTROL OF A SENSOR FOR DETECTING PARTICLES AND SENSOR FOR DETECTING PARTICLES
PROCÉDÉ POUR LE CONTRÔLE DU FONCTIONNEMENT D'UN CAPTEUR SERVANT À LA DÉTECTION DE PARTICULES ET CAPTEUR SERVANT À LA DÉTECTION DE PARTICULES

(30) Priorität: 21.06.2012 DE 102012210525
(43) Veröffentlichungstag der Anmeldung: 29.04.2015
(73) Patentinhaber: Robert Bosch GmbH, 70442 Stuttgart (DE)
(72) Erfinder: GAERTNER, Benjamin, 76149 Neureut (DE); KLENK, Mathias, 72074 Tuebingen (DE); HERWEG, Karola, 70469 Stuttgart (DE); BESSEN, Michael, 70190 Stuttgart (DE)
(86) Internationale Anmeldenummer: PCT/EP2013/062151
(87) Internationale Veröffentlichungsnummer: WO 2013/189806

(56) Entgegenhaltungen:
- EP-A2- 2 343 529
- DE-A1- 10 059 683
- DE-A1- 10 111 269
- DE-A1- 10 149 333
- DE-A1-102005 030 134
- DE-A1-102010 042 226
- DE-A1-102011 079 710
- FR-A1- 2 919 928
- US-A1- 2003 196 499
- US-A1- 2011 109 331
- US-A1- 2012 031 169

## Beschreibung

### Stand der Technik

Aus dem Stand der Technik sind zahlreiche Verfahren und Vorrichtungen zur Detektion von Teilchen, wie beispielsweise Ruß- oder Staubpartikeln, bekannt. Die Erfindung wird im Folgenden, ohne Beschränkung weiterer Ausführungsformen und Anwendungen, insbesondere unter Bezugnahme auf Sensoren zur Detektion von Teilchen beschrieben, insbesondere von Rußpartikeln in einem Abgasstrom einer Brennkraftmaschine.

Es ist aus der Praxis bekannt, mittels zweier Elektroden, die auf einer Keramik angeordnet sind, eine Konzentration von Teilchen, wie beispielsweise Ruß- oder Staubpartikeln, in einem Abgas zu messen. Dies kann beispielsweise durch eine Messung des elektrischen Widerstands des die beiden Elektroden trennenden keramischen Werkstoffs erfolgen. Derartige Sensoren werden beispielsweise in einem Abgasstrang einer Brennkraftmaschine, wie beispielsweise eines Verbrennungsmotors der Dieselbauart, eingesetzt. Üblicherweise befinden sich diese stromabwärts des Verbrennungsmotors bzw. des Dieselpartikelfilters. Ein steigendes Umweltbewusstsein und auch zum Teil bedingt durch gesetzliche Vorschriften muss zukünftig der Rußausstoß während des Fahrbetriebs eines Kraftfahrzeugs überwacht und die Funktionalität dieser Überwachung sichergestellt werden. Diese Art der Überwachung der Funktionalität wird allgemein als On-Bord-Diagnose bezeichnet. Darüber hinaus ist eine Beladungsprognose von Dieselpartikelfiltern notwendig, um eine hohe Systemsicherheit bei wenigen effizienten, kraftstoffsparenden Regenerationszyklen zu erreichen, um kostengünstigere Filtermaterialien, wie beispielsweise Cordierit, einsetzen zu können. Eine Möglichkeit hierzu bietet ein resistiver Rußsensor, der die Widerstandsänderung einer interdigitalen Elektrodenstruktur aufgrund von Rußanlagerung zur Detektion des Rußes heranzieht. Aufgrund seiner Funktionsweise ordnet sich der resistive Rußsensor bei den sammelnden Prinzipien ein. Derartige Rußsensoren sind beispielsweise aus der DE 101 49 333 A1 oder WO 2003/006976 A2 bekannt.

Bei derartigen resistiven Partikelsensoren für leitfähige Partikel sind zwei oder mehrere metallische Elektroden auf einem elektrisch isolierenden Substrat ausgebildet, wobei die sich unter Einwirkung einer elektrischen Messspannung anlagernden Teilchen, insbesondere Rußpartikel, die kammartig ineinander greifenden Elektroden kurzschließen und zwischen den Sensorelektroden ein abnehmender Widerstand bzw. ein zunehmender Strom bei konstanter angelegter Spannung messbar wird. Zur Regeneration des Sensorelements nach Rußanlagerung wird das Sensorelement mit Hilfe eines integrierten Heizelements freigebrannt. Die Auswertung des Sensorsignals erfolgt im System durch einen Vergleich von der aus einem Signalverhaltenmodell unter Einbeziehung des Rohemissionsmodells ermittelten Soll-Auslösezeit und der tatsächlichen Sensorauslösezeit.

Um im Feld die Funktionalität der Elektroden und somit des Sensors zu überwachen, wird an die Elektroden am Ende der Regeneration eine Messspannung angelegt. Dadurch entsteht ein lonenstrom, der zumeist durch Verunreinigungen in Form von Natrium bedingt ist. Überschreitet dieser einen bestimmten Schwellwert, so sind die Elektroden als intakt zu betrachten.

Trotz der zahlreichen Vorteile der aus dem Stand der Technik bekannten Verfahren und Vorrichtung zur Detektion von Teilchen beinhalten diese noch Verbesserungspotential. So ist die oben beschriebene Form der Eigendiagnose nur bedingt alterungsstabil. Bisher wird die Eigenleitfähigkeit der Elektrodenmesszelle in der Regel einmalig bei einer bestimmten Temperatur gemessen. Mit der Alterung des Sensors geht jedoch in der Regel eine Abnahme des Eigendiagnosestroms einher. Somit kann ab einem bestimmten Alterungszeitpunkt des Sensors der Eigendiagnosestrom den Schwellwert unterschreiten, ohne dass dies auf einen Defekt des Sensors zurückzuführen ist. Ein Sensor, bei dem ein Strom kleiner als 2 µA gemessen wird, gilt beispielsweise bislang in vielen Fällen als Ausschuss, da bis zu 1,5 µA auf Nebenschlüsse zurückgeführt werden können. Somit kann ab einem bestimmten Alterungszeitpunkt nicht mehr unterschieden werden, ob das Unterschreiten des Schwellwerts aufgrund der Alterung oder aufgrund eines Defekts verursacht ist.

Weitere Verfahren und Vorrichtung zur Detektion von Teilchen sind bekannt aus der US 2012/0031169 A1, DE 10 2011 079 710 A1, US 2011/0109331 A1 und EP 2 343 529 A2.

### Offenbarung der Erfindung

Es werden daher ein Verfahren zur Funktionskontrolle eines Sensors zur Detektion von Ruß, sowie ein Sensor zur Detektion von Ruß, vorgeschlagen, welche die Nachteile bekannter Verfahren und Vorrichtungen zumindest weitgehend vermeiden und bei denen beispielsweise Nebenschlüsse als Offset eliminiert werden können.

Das erfindungsgemäße Verfahren zur Funktionskontrolle eines Sensors zur Detektion von von Ruß gemäß Anspruch 1, wobei der Sensor mindestens zwei Messelektroden, die auf einem Substrat aus einem elektrisch isolierenden Werkstoff angeordnet sind, und ein Heizelement umfasst, umfasst die folgenden Schritte, bevorzugt in der folgenden Reihenfolge:
- Durchführen einer ersten Strom-Spannungsmessung bei einer ersten Temperatur zum Ermitteln einer ersten Messgröße,
- Durchführen einer zweiten Strom-Spannungsmessung bei einer zweiten Temperatur zum Ermitteln einer zweiten Messgröße und
- Bilden einer Differenz aus der ersten Messgröße und der zweiten Messgröße.

Die zweite Strom-Spannungsmessung wird erfindungsgemäß nur durchgeführt falls die erste Messgröße einen Schwellwert unterschreitet.

Die zweite Temperatur ist erfindungsgemäß niedriger als die erste Temperatur. Die zweite Temperatur kann um 80 °C bis 220 °C, bevorzugt um 100 °C bis 200 °C und noch bevorzugter um 120 °C bis 180 °C niedriger sein als die erste Temperatur, beispielsweise um 150 °C. Die zweite Strom-Spannungsmessung kann zeitlich nach der ersten Strom-Spannungsmessung durchgeführt werden. Die zweite Strom-Spannungsmessung kann beispielsweise 0,5 s bis 20,0 s nach der ersten Strom-Spannungsmessung durchgeführt werden. Bevorzugt kann die zweite Strom-Spannungsmessung 1,0 s bis 20,0 s, bevorzugt 1,0 s bis 15 s und noch bevorzugter von 1,0 bis 12 s zeitlich nach der ersten Strom-Spannungsmessung durchgeführt werden, beispielsweise innerhalb von 10 s nach der ersten Strom-Spannungsmessung.

Die erste Strom-Spannungsmessung und/oder die zweite Strom-Spannungsmessung können jeweils über einen Zeitraum von 100 ms bis 500 ms, bevorzugt von 200 ms bis 400 ms und noch bevorzugter von 250 ms bis 350 ms durchgeführt werden, beispielsweise 300 ms.

Die erste Strom-Spannungsmessung und/oder die zweite Strom-Spannungsmessung können jeweils einfach oder auch wiederholt durchgeführt werden. Die oben beschriebene Differenz kann dabei beispielsweise zwischen Messgrößen gebildet werden, die aus zeitlich benachbarten Strom-Spannungsmessungen ermittelt wurden, oder zwischen Messgrößen, die aus zeitlich nicht benachbarten Strom-Spannungsmessungen ermittelt wurden.

Als Schwellwert für eine Bestimmung der Funktionsfähigkeit des Sensors kann insbesondere eine Differenz zwischen der ersten Messgröße und der zweiten Messgröße von 0,10 µA bis 0,60 µA, bevorzugt von 0,15 µA bis 0,35 µA und noch bevorzugter von 0,20 µA bis 0,30 µA festgelegt werden, beispielsweise 0,25 µA. Die erste Temperatur kann im Wesentlichen konstant gehalten werden über einen Zeitraum von 20 s bis 80 s, bevorzugt von 30 s bis 60 s und noch bevorzugter von 40 s bis 50 s, beispielsweise 45 s, wobei die erste Strom-Spannungsmessung am Ende des Zeitraums gemessen wird. Ein Schwellwert für die erste Messgröße kann ein Strom von 1,5 µA bis 2,5 µA und noch bevorzugter von 1,7 µA bis 2,3 µA und noch bevorzugter von 1,8 µA bis 2,2 µA sein, beispielsweise 2,0 µA. Die erste Temperatur kann von 700 °C bis 860 °C, bevorzugt von 740 °C bis 820 °C und noch bevorzugter von 760 °C bis 800 °C sein, beispielsweise 785 °C, wobei die zweite Temperatur von 560 °C bis 700 °C, bevorzugt von 600 °C bis 660 °C und noch bevorzugter von 620 °C bis 640 °C ist, beispielsweise 635 °C. Die Strom-Spannungsmessung kann eine Messspannung von 7,0 V bis 10,0 V und bevorzugt von 7,5 V bis 9,0 V umfassen, beispielsweise 8,5 V.

Ein erfindungsgemäßer Sensor zur Detektion von Teilchen gemäß Anspruch 10, insbesondere von Ruß in einem Abgasstrom in einer Brennkraftmaschine, umfasst mindestens zwei Messelektroden, die auf einem Substrat aus einem elektrisch isolierenden Werkstoff angeordnet sind, ein Heizelement und eine Steuerung, wobei die Steuerung zum Ausführen eines Verfahrens nach einem der Ansprüche 1 bis 9 eingerichtet ist.

Die Messelektroden können insbesondere als Interdigitalelektroden ausgebildet sein, also als ineinander eingreifende Messelektroden, beispielsweise zwei oder mehr ineinander eingreifende Kammstrukturen.

Unter Teilchen im Sinne der vorliegenden Erfindung sind insbesondere elektrisch leitfähige Teilchen zu verstehen, wie beispielsweise Ruß oder Staub.

Unter Messelektroden sind im Rahmen der vorliegenden Erfindung Elektroden zu verstehen, die geeignet sind zur Messung eines Stroms oder einer elektrischen Spannung.

Unter einem elektrisch isolierenden Werkstoff ist im Rahmen der vorliegenden Erfindung jeder Werkstoff zu verstehen, der geeignet ist, einen Stromfluss zu verhindern, wie beispielsweise eine Keramik.

Unter einer Strom-Spannungsmessung ist im Rahmen der vorliegenden Erfindung eine Messung zu verstehen, bei der entweder an die Messelektroden eine bestimmte Spannung angelegt wird und ein Stromfluss zwischen den Messelektroden gemessen wird oder an die Messelektroden ein Strom angelegt wird und eine Spannung zwischen den Messelektroden gemessen wird. Eine Strom-Spannungsmessung kann insbesondere eine Widerstandsmessung sein, wobei ein Widerstand des durch die Messelektroden und das Substrat gebildeten Aufbaus gemessen werden kann. Es kann beispielsweise eine spannungsgesteuerte oder spannungsgeregelte Messung und/oder eine stromgesteuerte und/oder stromgeregelte Messung erfolgen. Das Anlegen des Stroms und/oder der Spannung können in Form eines kontinuierlichen Signals und/oder auch in Form eines gepulsten Signals erfolgen. So können beispielsweise eine Gleichspannung und/oder ein Gleichstrom angelegt werden und eine Stromantwort bzw. eine Spannungsantwort erfasst werden. Alternativ können eine gepulste Spannung und/oder ein gepulster Strom angelegt werden und eine Stromantwort bzw. eine Spannungsantwort erfasst werden.

Im Rahmen der vorliegenden Erfindung ist unter einer Messgröße eine durch die Strom-Spannungsmessung ermittelte Größe zu verstehen, die entsprechend ein elektrischer Strom oder eine elektrische Spannung sein kann. Auch ein daraus hergeleiteter elektrischer Widerstand kann als Messgröße verwendet werden.

Unter einem im Wesentlichen Konstanthalten einer Temperatur ist im Rahmen der vorliegenden Erfindung das Halten einer Temperatur auf einem bestimmten vorgegebenen Wert über einen bestimmten Zeitraum zu verstehen, der eine Abweichung von maximal 15 °C und bevorzugt von maximal 5 °C umfasst.

Unter dem Durchführen einer Strom-Spannungsmessung am Ende eines Zeitraums ist im Rahmen der vorliegenden Erfindung ein Durchführen der Strom-Spannungsmessung derart zu verstehen, dass die Strom-Spannungsmessung im Wesentlichen zeitgleich mit dem Zeitraum endet, d. h. mit einem zeitlichen Versatz von maximal 1 s.

Unter einer Steuerung ist im Rahmen der vorliegenden Erfindung jedes Gerät zu verstehen, das geeignet ist, das erfindungsgemäße Verfahren durchzuführen und dabei die entsprechenden Steuerungs- und/oder Regelungsvorgänge durchzuführen. Die Steuerung kann eine dem Sensor zugeordnete eigene Steuerung oder auch Teil einer Steuerung einer Brennkraftmaschine sein, wie beispielsweise Teil einer Motorsteuerung eines Verbrennungsmotors, insbesondere eines Dieselmotors.

Unter Interdigitalelektroden sind im Rahmen der vorliegenden Erfindung Elektroden zu verstehen, die so angeordnet sind, dass sie ineinander eingreifen, insbesondere kammförmig.

Im Rahmen der vorliegenden Erfindung wird somit eine Änderung der Betriebsstrategie und Anpassung der Software der Betriebselektronik einer Brennkraftmaschine vorgeschlagen, bei der die Lebensdauer der Elektrodeneigendiagnose deutlich verbessert werden kann. Insbesondere werden durch eine zweimalige Messung der Eigenleitfähigkeit der Elektrodenmesszelle bei verschiedenen Temperaturen und Differenzbetrachtung Nebenschluss-Offsetströme eliminiert und die Bewertungsgrenze kann herabgesetzt werden. Im Vergleich zu dem oben beschriebenen Stand der Technik wird nun als Bewertungskriterium die Differenz zwischen zwei Strom- oder Spannungswerten herangezogen. Dadurch, dass bei einer Differenzbildung die Nebenschlüsse als Offset eliminiert werden können, kann die Bewertungsschwelle für die Funktionalität des Sensors deutlich herabgesetzt werden, beispielsweise auf 0,25 µA. Eine Alterung der Sensoren und eine damit verbundene Abnahme des Eigendiagnosestroms führt damit erst deutlich später zu einem Sensorausfall.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
- Fig. 1: eine Explosionsdarstellung eines Sensors zur Detektion von Teilchen; und
- Fig. 2: ein Zeitdiagramm des erfindungsgemäßen Verfahrens.

### Ausführungsformen der Erfindung

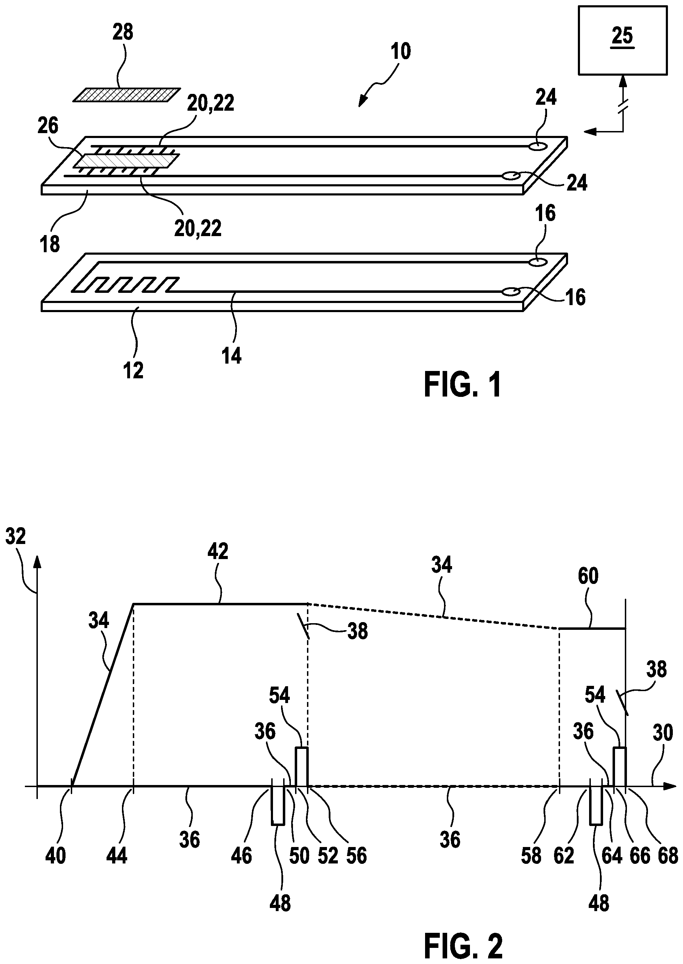

Fig. 1 zeigt einen Sensor 10 zur Detektion von Teilchen, insbesondere von Ruß, in einem Gasstrom, wie beispielsweise einem Abgasstrom einer Brennkraftmaschine, der zum Einbau in einen Abgasstrang eines Kraftfahrzeugs dient. Beispielsweise ist der Sensor 10 als Rußsensor ausgebildet und bevorzugt stromabwärts eines Rußfilters eines Kraftfahrzeugs mit einem Dieselverbrennungsmotor angeordnet.

Der Sensor 10 umfasst eine plattenförmige Trägerschicht 12, die zumindest teilweise aus einem elektrisch isolierenden Werkstoff hergestellt ist, wie beispielsweise aus einer Keramik wie Aluminiumoxid. In die Trägerschicht 12 ist ein Heizelement 14 integriert, das über Kontaktierungen 16 mit einer geeigneten Spannungsquelle verbindbar ist und zum Freibrennen des Sensors 10 von gegebenenfalls abgelagerten Teilchen, wie Rußpartikeln, dient.

Auf der Trägerschicht 12 ist ein plattenförmiges Substrat 18 angeordnet, das zumindest teilweise aus einem elektrisch isolierenden Werkstoff hergestellt ist, wie beispielsweise einer Keramik wie Aluminiumoxid. Auf dem Substrat 18 ist eine Struktur aus zwei Messelektroden 20 angeordnet. Beispielsweise sind die Messelektroden 20 als Interdigitalelektroden 22 ausgebildet, so dass sie kammförmig ineinandergreifen. Die Messelektroden 20 sind über Kontaktierungen 24 mit einer Steuerung 25 verbindbar.

In dem Bereich, in dem die Messelektroden 20 kammförmig ineinander greifen, können die Messelektroden 20 zumindest teilweise von einem Dielektrikum 26 überdeckt sein, so dass die Messelektroden 20 als Elektroden eines Kondensators mit messbarer Kapazität dienen können. Das Dielektrikum 26 kann wiederum mit einer Schutzschicht 28 versehen sein, so dass es gegenüber dem umgebenden Medium abgetrennt ist, wodurch eine Degeneration des Dielektrikums 26 ausgeschlossen ist.

Der Sensor 10 kann ferner ein Gehäuse umfassen, das den in Fig. 1 dargestellten Aufbau umgibt und aus Gründen der Vereinfachung der Erläuterung des Aufbaus des Sensors 10 in Fig. 1 nicht gezeigt ist. Beispielsweise kann das Gehäuse als Fanghülse ausgebildet sein, die in einem oberhalb der Messelektroden 20 liegenden Bereich mit einer Öffnung versehen ist und zur Beruhigung eines in dem Abgasstrang strömenden Gasstroms dient, so dass sich Rußpartikel bzw. sonstige, in dem Gasstrom enthaltene Teilchen bevorzugt im Bereich der Messelektroden 20 ablagern.

Der Sensor 10 nach der Fig. 1 kann wie folgt arbeiten. Wenn sich auf dem Substrat 18 Ruß bzw. sonstige elektrisch leitenden Teilchen ablagern, so reduziert sich ein elektrischer Widerstand zwischen den beiden Messelektroden 20. Durch Messung der Impedanz zwischen den beiden Messelektroden 20 ergibt sich ein für ein so genanntes RC-Glied typisches Verhalten. Dies bedeutet, dass die Ruß- bzw. Teilchenkonzentration in dem betreffenden Abgas anhand der zeitlichen Änderung des Widerstandsanteils des RC-Gliedes bestimmt werden kann.

Zur Regeneration des Sensors 10 werden die angelagerten Teilchen nach gewisser Zeit mittels des in die Trägerschicht 12 integrierten Heizelements 14 abgebrannt. Bei funktionstüchtigem Sensor 10 sollte nach diesem so genannten Ausheizen der Widerstand zwischen den Messelektroden 20 deutlich ansteigen und bevorzugt gegen Unendlich gehen. Da die Arbeitsweise des Sensors 10 Detektion der Teilchenkonzentration an sich bekannt ist, beispielsweise aus dem oben genannten Stand der Technik der WO 2003/006976 A2, wird an dieser Stelle nicht näher auf die gewöhnliche Arbeitsweise des Sensors 10 eingegangen und der Inhalt des oben genannten Stands der Technik, der die Beschreibung der Funktionsweise des Sensors 10 betrifft, ist durch Verweis hierin vollständig eingeschlossen. Stattdessen wird nun das erfindungsgemäße Verfahren zur Funktionskontrolle des Sensors 10 beschrieben. Das Verfahren kann beispielsweise von der oben genannten Steuerung 25 durchgeführt werden. Insbesondere wird das Verfahren anhand der Fig. 2 beschrieben.

Insbesondere ist in Fig. 2 die Zeit auf der X-Achse 30 dargestellt und auf der Y-Achse 32 der Verlauf bestimmter Größen, wie einer Temperatur 34, einer elektrischen Spannung 36 und eines elektrischen Stroms 38 über die Zeit dargestellt. Fig. 2 zeigt beispielsweise das oben beschriebene Freibrennen oder Ausheizen des Sensors 10. Dazu wird der Sensor 10 mittels des Heizelements 14 erhitzt, wie beispielsweise anhand des Verlaufs der Temperatur 34 ab einem ersten Zeitpunkt 40 erkennbar ist. Der Sensor 10 wird erhitzt bis zum Erreichen einer ersten Temperatur 42 zu einem zweiten Zeitpunkt 44 an. Die erste Temperatur 42 wird ab dem zweiten Zeitpunkt 44 im Wesentlichen konstant gehalten über einen Zeitraum von 20 s bis 80 s, bevorzugt von 30 s bis 60 s und noch bevorzugter von 40 s bis 50 s, beispielsweise 45 s. Die erste Temperatur 42 kann beispielsweise von 700 °C bis 860 °C, bevorzugt von 740 °C bis 820 °C und noch bevorzugter von 760 °C bis 800 °C sein, beispielsweise 785 °C.

Ferner ist in Fig. 2 der Verlauf der an die Messelektroden 20 angelegten elektrischen Spannung 36 dargestellt. Wie aus Fig. 2 erkennbar ist, ist die Spannung zwischen dem ersten Zeitpunkt 40 und dem zweiten Zeitpunkt 44 konstant 0 V. Zu einem dritten Zeitpunkt 46 wird an die Messelektroden 20 eine negative Spannung 48 von -7,5 V bis -9,0 V angelegt, beispielsweise -8,5 V, die für eine Umpolung aufgrund der Ladungsträger der Keramik des Substrats 18 verwendet wird. Die negative Spannung 48 wird bis zu einem vierten Zeitpunkt 50 konstant gehalten. Der Zeitraum zwischen dem dritten Zeitpunkt 46 und dem vierten Zeitpunkt 50 ist beispielsweise 300 ms. Zwischen dem vierten Zeitpunkt 50 und einem kurz darauf folgenden fünften Zeitpunkt 52 liegt wieder die Spannung 36 von 0 V an.

Ab dem fünften Zeitpunkt 52 wird eine erste Strom-Spannungsmessung zur Ermittlung einer ersten Messgröße durchgeführt. Bei diesem Ausführungsbeispiel wird die Strom-Spannungsmessung derart durchgeführt, dass an die Messelektroden 20 eine konstante Messspannung 54 angelegt wird und als Messgröße der zwischen den Messelektroden 20 fließende elektrische Strom 38 ermittelt wird. Es ist jedoch alternativ möglich, einen Messstrom an die Messelektroden 20 anzulegen und die Spannung zu messen. Die Messspannung 54 kann von 7,0 V bis 10,0 V und bevorzugt von 7,5 V bis 9,0 V sein, beispielsweise 8,5 V. Die erste Strom-Spannungsmessung wird bei der ersten Temperatur 42 bis zu einem sechsten Zeitpunkt 56 durchgeführt. Ab dem sechsten Zeitpunkt 56 liegt an den Messelektroden wieder eine Spannung 36 von 0 V an. Mit anderen Worten wird die erste Strom-Spannungsmessung am Ende des Zeitraums durchgeführt, über den die erste Temperatur 42 konstant gehalten wird. Der Zeitraum zwischen dem fünften Zeitpunkt 52 und dem sechsten Zeitpunkt 56, über den die erste Strom-Spannungsmessung durchgeführt wird, kann von 100 ms bis 500 ms, bevorzugt von 200 ms bis 400 ms und noch bevorzugter von 250 ms bis 350 ms durchgeführt werden, beispielsweise 300 ms. Fig. 2 zeigt dabei, dass bei Anlegen der Messspannung 54 der Strom 38 als erste Messgröße in einer sinkenden Form aufgrund einer Wanderung von Ladungsträgern erfasst wird. Als erste Messgröße kann daher der über den Zeitraum der ersten Strom-Spannungsmessung zeitlich gemittelte Stromwert verwendet werden.

Überschreitet die erste Messgröße in Form des elektrischen Stroms 38 einen Schwellwert der ersten Messgröße von 1,5 µA bis 2,4 µA und bevorzugt von 1,7 µA bis 2,3 µA und noch bevorzugter von 1,8 µA bis 2,2 µA, beispielsweise 2,0 µA, so zeigt dies bereits die Funktionalität des Sensors 10 an. In diesem Fall sind weitere Messungen nicht zwingend erforderlich. Unterschreitet die erste Messgröße den Schwellwert für die erste Messgröße, wird das erfindungsgemäße Verfahren fortgesetzt. Dazu wird die Temperatur 34 zwischen dem sechsten Zeitpunkt 56 und einem siebten Zeitpunkt 58 deutlich abgesenkt auf eine zweite Temperatur 60. Die zweite Temperatur 60 ist beispielsweise von 80 °C bis 220 °C, bevorzugt von 100 °C bis 200 °C und noch bevorzugter von 120 °C bis 180 °C niedriger als die erste Temperatur 42, beispielsweise 150 °C niedriger als die erste Temperatur 42. Beispielsweise ist die zweite Temperatur 58 von 560 °C bis 700 °C, bevorzugt von 600 °C bis 660 °C und noch bevorzugter von 620 °C bis 640 °C, beispielsweise 635 °C. Die zweite Temperatur 60 wird ab dem siebten Zeitpunkt 58 ebenfalls konstant gehalten.

Zu einem achten Zeitpunkt 62 wird an die Messelektroden 20 wiederum die negative Spannung 48 von -8,5 V angelegt, die für eine Umpolung aufgrund der Ladungsträger der Keramik des Substrats 18 verwendet wird. Die negative Spannung 48 wird bis zu einem neunten Zeitpunkt 64 konstant gehalten. Der Zeitraum zwischen dem achten Zeitpunkt 62 und dem neunten Zeitpunkt 64 ist beispielsweise 300 ms. Zwischen dem neunten Zeitpunkt 64 und einem kurz darauf folgenden zehnten Zeitpunkt 66 liegt wieder die Spannung 36 von 0 V an.

Ab dem zehnten Zeitpunkt 66 wird eine zweite Strom-Spannungsmessung zur Ermittlung einer zweiten Messgröße durchgeführt. Bei diesem Ausführungsbeispiel wird die zweite Strom-Spannungsmessung analog de ersten Strom-Spannungsmessung derart durchgeführt, dass an die Messelektroden 20 die konstante Messspannung 54 von 8,5 V angelegt wird und als zweite Messgröße der zwischen den Messelektroden 20 fließende elektrische Strom 38 ermittelt wird. Die zweite Strom-Spannungsmessung wird jedoch bei der zweiten Temperatur 60 bis zu einem elften Zeitpunkt 68 durchgeführt. Der Zeitraum zwischen dem zehnten Zeitpunkt 66 und dem elften Zeitpunkt 68, über den die zweite Strom-Spannungsmessung durchgeführt wird, kann von 100 ms bis 500 ms, bevorzugt von 200 ms bis 400 ms und noch bevorzugter von 250 ms bis 350 ms durchgeführt werden, beispielsweise 300 ms. Fig. 2 zeigt dabei, dass bei Anlegen der Messspannung 54 der Strom 38 als zweite Messgröße in einer sinkenden Form aufgrund einer Wanderung von Ladungsträgern erfasst wird. Als zweite Messgröße kann daher der über den Zeitraum der zweiten Strom-Spannungsmessung zeitlich gemittelte Stromwert verwendet werden. Aufgrund der mit sinkender Temperatur abnehmenden Leitfähigkeit ist der Strom 38 bei der zweiten Strom-Spannungsmessung niedriger als der Strom 38 bei der ersten Strom-Spannungsmessung.

Der Zeitraum für die Messung der zweiten Strom-Spannungsmessung ist identisch mit dem Zeitraum der ersten Strom-Spannungsmessung. Die zweite Strom-Spannungsmessung wird dabei 1,0 s bis 20,0 s, bevorzugt von 1,0 s bis 15 s, noch bevorzugter von 1,0 bis 12 s zeitlich nach der ersten Strom-Spannungsmessung durchgeführt, beispielsweise innerhalb von 10 s nach der ersten Strom-Spannungsmessung. Der Zeitraum hängt unter anderem von der Geschwindigkeit der Abkühlung des Sensors 10 ab. Nachdem die zweite Messgröße durch die zweite Strom-Spannungsmessung ermittelt wurde, wird eine Differenz zwischen der ersten Messgröße und der zweiten Messgröße gebildet, d. h. der ermittelte Stromwert 38 der zweiten Strom-Spannungsmessung wird von dem ermittelten Stromwert 38 der ersten Strom-Spannungsmessung subtrahiert. Als Bemessungsgrundlage, basierend auf der auf eine Funktionalität des Sensors 10 geschlossen wird, wird nun eine Differenz zwischen der ersten Messgröße und der zweiten Messgröße mit einem Schwellwert von beispielsweise 0,10 µA bis 0,60 µA, bevorzugt von 0,15 µA bis 0,35 µA und noch bevorzugter von 0,20 µA bis 0,30 µA festgelegt, beispielsweise 0,25 µA. Wird der Schwellwert unterschritten, so wird auf einen Defekt des Sensors 10 geschlossen. Wird dieser Schwellwert überschritten, kann auf die Funktionalität des Sensors 10 geschlossen werden. Durch die Differenzbildung werden Nebenschlüsse als Offset eliminiert. Entsprechend führt eine Alterung des Sensors 10 und eine damit verbundene Abnahme des Diagnosestroms erst zu einem deutlich späteren Ausfall des Sensors 10.

## Patentansprüche

1. Verfahren zur Funktionskontrolle eines Sensors (10) zur Detektion von Ruß, wobei der Sensor (10) mindestens zwei Messelektroden (20), die auf einem Substrat (18) aus einem elektrisch isolierenden Werkstoff angeordnet sind, und ein Heizelement (14) umfasst, wobei das Verfahren die folgenden Schritte umfasst:
- Konstanthalten einer ersten Temperatur, die zwischen 700°C und 860°C ist, über einen Zeitraum von 20s - 80s und Durchführen einer ersten Strom-Spannungsmessung bei der ersten Temperatur (42) am Ende des Zeitraums zum Ermitteln einer ersten Messgröße
- Aus einem Überschreiten der ersten Messgröße eines Schwellwerts wird geschlossen, dass der Sensor funktional ist
- Nur falls die erste Messgröße den Schwellwert unterschreitet:
Durchführen einer zweiten Strom-Spannungsmessung zeitlich nachfolgend der ersten Strom-Spannungsmessung bei einer zweiten Temperatur (60) zum Ermitteln einer zweiten Messgröße, wobei die zweite Temperatur (60) niedriger als die erste Temperatur (42) ist und von 540 °C bis 700 °C ist, Bilden einer Differenz aus der ersten Messgröße und der zweiten Messgröße, wobei aus dem Überschreiten der Differenz eines weiteren Schwellwerts geschlossen wird, dass der Sensor funktional ist und aus dem Unterschreiten der Differenz des weiteren Schwellwerts geschlossen wird, dass der Sensor defekt ist.

2. Verfahren nach Anspruch 1, wobei die zweite Temperatur (60) von 80 °C bis 220 °C, bevorzugt von 100 ° bis 200 °C und noch bevorzugter von 120 °C bis 180 °C niedriger als die erste Temperatur (42) ist.

3. Verfahren nach dem vorhergehenden Anspruch, wobei die zweite Strom-Spannungsmessung von 1,0 s bis 20,0 s, bevorzugt von 1,0 s bis 15 s und noch bevorzugter von 1,0 bis 12 s zeitlich nach der ersten Strom-Spannungsmessung durchgeführt wird.

4. Verfahren nach einem der vorhergehenden Ansprüche, wobei die erste Strom-Spannungsmessung und/oder die zweite Strom-Spannungsmessung über eine Zeitraum von 100 ms bis 500 ms, bevorzugt von 200 ms bis 400 ms und noch bevorzugter von 250 ms bis 350 ms durchgeführt werden.

5. Verfahren nach einem der vorhergehenden Ansprüche, wobei als Schwellwert für eine Bestimmung der Funktionsfähigkeit des Sensors (10) eine Differenz zwischen der ersten Messgröße und der zweiten Messgröße von 0,10 µA bis 0,60 µA, bevorzugt von 0,15 µA bis 0,35 µA und noch bevorzugter von 0,20 µA bis 0,30 µA festgelegt wird.

6. Verfahren nach einem der vorhergehenden Ansprüche, wobei die erste Temperatur (42) im Wesentlich konstant gehalten wird über von 30 s bis 60 s und bevorzugt von 40 s bis 50 s, wobei die erste Strom-Spannungsmessung am Ende des Zeitraums gemessen wird

7. Verfahren nach einem der vorhergehenden Ansprüche, wobei ein Schwellwert für die erster Messgröße ein Strom (38) von 1,5 µA bis 2,5 µA und bevorzugt von 1,7 µA bis 2,3 µA und noch bevorzugter von 1,8 µA bis 2,2 µA ist.

8. Verfahren nach einem der vorhergehenden Ansprüche, wobei die erste Temperatur (42) von 740 °C bis 820 °C und bevorzugter von 760 °C bis 800 °C ist, wobei die zweite Temperatur (60) von 560 °C bis 700 °C, bevorzugt von 600 °C bis 660 °C und noch bevorzugter von 620 °C bis 640 °C ist.

9. Verfahren nach einem der vorhergehenden Ansprüche, wobei die Strom-Spannungsmessung eine Messspannung (54) von 7,0 V bis 10,0 V und bevorzugt von 7,5 V bis 9,0 V umfasst.

10. Sensor (10) zur Detektion von Teilchen, insbesondere von Ruß in einem Abgasstrom einer Brennkraftmaschine, wobei der Sensor (10) mindestens zwei Messelektroden (20), die auf einem Substrat (18) aus einem elektrisch isolierenden Werkstoff angeordnet sind, ein Heizelement (14) und eine Steuerung (25) umfasst, wobei die Steuerung (25) zum Ausführen eines Verfahrens nach einem der vorhergehenden Ansprüche eingerichtet ist.

11. Sensor (10) nach dem vorhergehenden Anspruch, wobei die Messelektroden (20) als Interdigitalelektroden (22) ausgebildet sind.

## Claims

1. Method for monitoring the function of a sensor (10) for detecting soot, wherein the sensor (10) comprises at least two measurement electrodes (20) arranged on a substrate (18) composed of an electrically insulating material, and a heating element (14), wherein the method comprises the following steps:
- keeping constant a first temperature, which is between 700°C and 860°C, over a star of 20 s - 80 s and carrying out a first current-voltage measurement at the first temperature (42) at the end of the star in order to determine a first measurement variable
- from a threshold value being exceeded by the first measurement variable, it is deduced that the sensor is functional,
- only if the first measurement variable falls below the threshold value:
carrying out a second current-voltage measurement chronologically after the first current-voltage measurement at a second temperature (60) in order to determine a second measurement variable, wherein the second temperature (60) is lower than the first temperature (42) and is from 540°C to 700°C, forming a difference between the first measurement variable and the second measurement variable, wherein it is deduced from a further threshold value being exceeded by the difference that the sensor is functional, and it is deduced from the difference falling below the further threshold value that the sensor is defective.

2. Method according to Claim 1, wherein the second temperature (60) is from 80°C to 220°C, preferably from 100°C to 200°C and even more preferably from 120°C to 180°C, lower than the first temperature (42).

3. Method according to the preceding claim, wherein the second current-voltage measurement is carried out from 1.0 s to 20.0 s, preferably from 1.0 s to 15 s and even more preferably from 1.0 s to 12 s, chronologically after the first current-voltage measurement.

4. Method according to any of the preceding claims, wherein the first current-voltage measurement and/or the second current-voltage measurement are/is carried out over a star of 100 ms to 500 ms, preferably of 200 ms to 400 ms and even more preferably of 250 ms to 350 ms.

5. Method according to any of the preceding claims, wherein a difference between the first measurement variable and the second measurement variable of 0.10 µA to 0.60 µA, preferably of 0.15 µA to 0.35 µA and even more preferably of 0.20 µA to 0.30 µA, is defined as a threshold value for ascertaining the functionality of the sensor (10).

6. Method according to any of the preceding claims, wherein the first temperature (42) is kept substantially constant over from 30 s to 60 s, and preferably from 40 s to 50 s, wherein the first current-voltage measurement is measured at the end of the star.

7. Method according to any of the preceding claims, wherein a threshold value for the first measurement variable is a current (38) of 1.5 µA to 2.5 µA and preferably of 1.7 µA to 2.3 µA and even more preferably of 1.8 µA to 2.2 µA.

8. Method according to any of the preceding claims wherein the first temperature (42) is from 740°C to 820°C and more preferably from 760°C to 800°C, wherein the second temperature (60) is from 560°C to 700°C, preferably from 600°C to 660°C and even more preferably from 620°C to 640°C.

9. Method according to any of the preceding claims, wherein the current-voltage measurement comprises a measurement voltage (54) of 7.0 V to 10.0 V and preferably of 7.5 V to 9.0 V.

10. Sensor (10) for detecting particles, in particular soot in an exhaust gas flow of an internal combustion engine, wherein the sensor (10) comprises at least two measurement electrodes (20) arranged on a substrate (18) composed of an electrically insulating material, a heating element (14) and a controller (25), wherein the controller (25) is configured for carrying out a method according to any of the preceding claims.

11. Sensor (10) according to the preceding claim, wherein the measurement electrodes (20) are embodied as interdigital electrodes (22).

## Revendications

1. Procédé pour le contrôle du fonctionnement d'un capteur (10) servant à la détection de suie, dans lequel le capteur (10) comprend au moins deux électrodes de mesure (20) qui sont disposées sur un substrat (18) en un matériau électriquement isolant, et un élément chauffant (14), le procédé comprenant les étapes suivantes :
- maintenir constante une première température, qui est comprise entre 700 °C et 860 °C, pendant une période de 20 s à 80 s et exécuter une première mesure courant-tension à la première température (42) à la fin de la période de temps pour déterminer une première grandeur de mesure,
- lorsque la première grandeur de mesure dépasse une valeur de seuil, il en est conclu que le capteur est fonctionnel,
- uniquement dans le cas où la première grandeur de mesure s'abaisse en-dessous de la valeur de seuil :
exécuter une deuxième mesure courant-tension après la première mesure courant-tension à une deuxième température (60) pour la détermination d'une deuxième grandeur de mesure, dans lequel la deuxième température (60) est inférieure à la première température (42) et est comprise entre 540 °C et 700 °C, établir une différence entre la première grandeur de mesure et la deuxième grandeur de mesure, dans lequel, lorsque la différence dépasse une autre valeur de seuil, il en est conclu que le capteur est fonctionnel et lorsque la différence s'abaisse en-dessous de l'autre valeur de seuil, il en est conclu que le capteur est défectueux.

2. Procédé selon la revendication 1, dans lequel la deuxième température (60) est inférieure à la première température (42) de 80 °C à 220 °C, de préférence de 100 °C à 200 °C et encore plus préférablement de 120 °C à 180 °C.

3. Procédé selon la revendication précédente, dans lequel la deuxième mesure courant-tension est exécutée de manière décalée dans le temps de 1,0 à 20,0 s, de préférence de 1,0 à 15 s et encore plus préférablement de 1,0 à 12 s après la première mesure de courant-tension.

4. Procédé selon l'une des revendications précédentes, dans lequel la première mesure courant-tension et/ou la deuxième mesure courant-tension est effectuée au cours d'une période de temps de 100 ms à 500 ms, de préférence de 200 ms à 400 ms et encore plus préférablement de 250 ms à 350 ms.

5. Procédé selon l'une des revendications précédentes, dans lequel une différence entre la première grandeur de mesure et la deuxième grandeur de mesure de 0,10 µA à 0,60 µA, de préférence de 0,15 µA à 0,35 µA et encore plus préférablement de 0,20 µA à 0,30 µA est établie en tant que valeur de seuil pour la détermination de la fonctionnalité du capteur (10).

6. Procédé selon l'une des revendications précédentes, dans lequel la première température (42) est maintenue sensiblement constante sur une période de 30 s à 60 s et de préférence de 40 s à 50 s, dans lequel la première mesure courant-tension est effectuée à la fin de la période de temps.

7. Procédé selon l'une des revendications précédentes, dans lequel une valeur de seuil pour la première grandeur de mesure est un courant (38) de 1,5 µA à 2,5 µA et de préférence de 1,7 µA à 2,3 µA et encore plus préférablement de 1,8 µA à 2,2 µA.

8. Procédé selon l'une des revendications précédentes, dans lequel la première température (42) est de 740 °C à 820 °C et plus préférablement de 760 °C à 800 °C, dans lequel la deuxième température (60) est de 560 °C à 700 °C, de préférence de 600 °C à 660 °C et encore plus préférablement de 620 °C à 640 °C.

9. Procédé selon l'une des revendications précédentes, dans lequel la mesure courant-tension comprend une tension de mesure (54) de 7,0 V à 10,0 V et de préférence de 7,5 V à 9,0 V.

10. Capteur (10) servant à la détection de particules, en particulier de suie, dans un flux de gaz d'échappement d'un moteur à combustion interne, le capteur (10) comprenant au moins deux électrodes de mesure (20) disposées sur un substrat (18) en un matériau électriquement isolant, un élément chauffant (14) et une commande (25), dans lequel la commande (25) est conçue pour exécuter un procédé selon l'une des revendications précédentes.

11. Capteur (10) selon la revendication précédente, dans lequel les électrodes de mesure (20) sont réalisées sous forme d'électrodes interdigitées (22).
